(19) Europäisches Patentamt
European Patent Office
Office européen des brevets

(11) Veröffentlichungsnummer: **0 263 356 B1**

(12) **EUROPÄISCHE PATENTSCHRIFT**

(45) Veröffentlichungstag der Patentschrift: **02.10.91**

(51) Int. Cl.$^5$: **C07C 311/15**

(21) Anmeldenummer: **87113874.9**

(22) Anmeldetag: **23.09.87**

# (54) Verfahren zur Herstellung von Hydroxynaphthalin-sulfonsäureamiden.

(30) Priorität: **04.10.86 DE 3633906**

(43) Veröffentlichungstag der Anmeldung:
**13.04.88 Patentblatt 88/15**

(45) Bekanntmachung des Hinweises auf die Patenterteilung:
**02.10.91 Patentblatt 91/40**

(84) Benannte Vertragsstaaten:
**CH DE FR GB LI**

(56) Entgegenhaltungen:
**EP-A- 0 017 699**

**BERICHTE DER DEUTSCHEN CHEMISCHEN GESELLSCHAFT, Band 58, Nr. 7, 8. Juli 1925, Seiten 1211-1218,Verlag Chemie GmbH, Berlin, DE; K. DZIEWONSKI et al.: "Über beta-Methyl-naphthalin-Derivate"**

(73) Patentinhaber: **BAYER AG**
**W-5090 Leverkusen 1 Bayerwerk(DE)**

(72) Erfinder: **Bussmann, Werner, Dr.**
**Walter-Flex-Strasse 32**
**W-5090 Leverkusen(DE)**
Erfinder: **Leverenz, Klaus, Dr.**
**Heymannstrasse 44**
**W-5090 Leverkusen(DE)**

**EP 0 263 356 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein Verfahren zur Herstellung von Hydroxynaphthalinsulfonsäureamiden der Formel

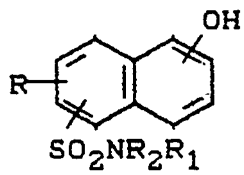

(I)

worin
R, $R_1$, $R_2$ = H oder Substituent
und deren Salzen, insbesondere deren Alkalisalzen, dadurch gekennzeichnet, daß man Disulfonamide der Formel

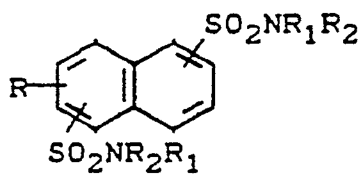

(II)

worin
R, $R_1$, $R_2$ = H, Substituent
bei etwa 180 bis 250 °C mit Alkalien behandelt.

Verbindungen des Typs (II) erhält man beispielsweise durch Umsetzung der Naphthalindisulfochloride

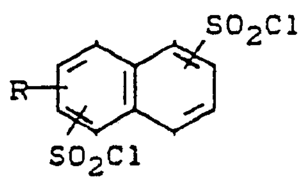

(III)

worin
R = H, Substituent
mit dem entsprechenden Amin $HNR_1R_2$ bzw. Ammoniak.

Alternativ kann man auch Naphthalinsulfonsäureanhydride (beispielsweise beschrieben in DE-A 28 53 335) mit Ammoniak bzw. einem Amin $HNR_1R_2$ umsetzen.

Die Umsetzung mit Alkalien erfolgt bevorzugt in wäßrigem Medium, das gegebenenfalls gegen Alkalihydrolyse inerte, wassermischbare Lösungsmittel enthalten kann, bei vorzugsweise 200 bis 230 °C.

Als Alkalien kommen insbesondere Alkali- und Erdalkalihydroxide in Frage, insbesondere Natrium- oder Kaliumhydroxid. Der pH-Bereich für die Umsetzung liegt bei etwa 12 bis 14.

Die Alkalihydroxide werden in Mengen von etwa 1 bis 10 Mol pro Mol (II) eingesetzt.

Geeignete Substituenten R sind H, $SO_3H$, Sulfonamid oder gegebenenfalls substituiertes Alkyl. Alkyl ist vorzugsweise gegebenenfalls substituiertes $C_1$-$C_4$-Alkyl.

Die Reste $R_1$ und $R_2$ können gleich oder verschieden sein. Geeignete Substituenten sind neben Wasserstoff beispielsweise gegebenenfalls substituiertes $C_1$-$C_4$-Alkyl oder gegebenenfalls substituiertes Phenyl insbesondere gegebenenfalls durch Nitro, Sulfo, CN, Halogen wie Cl oder Br substituiertes Phenyl.

Geeignete Ausgangsverbindungen (III) sind beispielsweise 1,5-Naphthalindisulfonsäurechlorid, 2,6-Naphthalindisulfonsäurechlorid, Armstrongsäureanhydrid (gemäß DE-A 28 53 337).

Eine bevorzugte Ausgestaltung des erfindungsgemäßen Verfahrens besteht darin, daß man die Verbindung (III) mit Ammoniak oder einem geeigneten Amin umsetzt, die Sulfonamide isoliert und in wäßrigem

oder wäßrig-organischem Medium im stark alkalischen Bereich unter Druck mit Alkali behandelt.

Die Verbindungen (I) sind bekannt. Sie werden beispielsweise als Kupplungskomponenten für die Herstellung von Azofarbstoffen eingesetzt.

Beispiel 1

5-Hydroxy-1-naphthalin-sulfonsäureamid

14,3 g (50 mmol) Naphthalin-1,5-disulfonsäureamid werden in 150 ml Wasser suspendiert und durch Zugabe von 100 ml 40 %iger Natronlauge in Lösung gebracht. Die Mischung wird in einem Stahlautoklaven auf 220° C gehalten,
bis vollständige Umsetzung eingetreten ist (10 bis 15 Stunden). Die so erhaltene Lösung wird von Verunreinigungen durch Filtration befreit und unter Rühren mit 150 ml 25 %iger wäßriger Schwefelsäure versetzt. Dabei fällt das Reaktionsprodukt in gut filtrierbarer Form aus. Man rührt noch 1 Stunde bei ca. 90° C um das Schwefeldioxid zu entfernen, rührt dann bis die Mischung abgekühlt ist und saugt ab.
Es werden 9,80 g (87 %) eines farblosen bis grauen Rohprodukts erhalten. Schmelzpunkt 264° C (Lit. 263), keine Schmelzpunktdepression beim Mischen mit einer authentischen Probe die aus Azurinsäure gewonnen wurde. Dünnschichtchromatographisch identisch mit Vergleich; gibt mit diazotiertem p-Nitroanilin einen roten Farbstoff. NMR-Daten: siehe Tabelle 1

Beispiel 2

6-Hydroxy-2-naphthalinsulfonsäureamid

28,6 g (0,1 mol) Naphthalin-2,6-disulfonsäureamid werden in 250 ml 10 %iger wäßriger Natronlauge gelöst. Man rührt 10 Stunden bei 220° C (23 bar)[1]. Die Reaktionsmischung wird durch Filtration von Verunreinigungen geklärt und der pH-Wert mit 50 ml 25 %iger Schwefelsäure auf 5,5 eingestellt, worauf das Produkt kristallin ausfällt. Dabei erwärmt sich die Mischung auf ca. 40° C. Es wird noch 1 Stunde kaltgerührt und der kristalline Niederschlag abgesaugt und im Vakuum bei 50° C getrocknet, 7,93 g (35,6 % der Theorie). Nach Einengen der Mutterlauge werden weitere 17,9 g durch Salz verunreinigtes Produkt erhalten.

1) Die Umsetzung wird chromatographisch auf Vollständigkeit geprüft, evtl. muß die Reaktionszeit verlängert werden.

Beispiel 3

5-Hydroxy-N-methyl-1-naphthalin-sulfonsäureamid

20,94 g (66,7 mmol) Di-(N-methyl)-1,5-naphthalin-disulfonsäureamid werden mit 150 ml Wasser und 100 ml 40 %iger wäßriger Natronlauge 10 Stunden bei 230° C gerührt (26 bar). Wenn das Chromatogramm kein Ausgangsprodukt mehr anzeigt wird die Reaktionsmischung geklärt und abgekühlt. Der pH wird durch Zugabe von Eisessig auf 6 eingestellt, worauf das Produkt ausfällt. Der kristalline Niederschlag wird abgesaugt und getrocknet (4,4 g). Aus der Mutterlauge kristallisieren nach mehrstündigem Stehen noch weitere 6,0 g Produkt aus. Zusammen: 10,4 g (65,8 % der Theorie)
NMR (siehe Tabelle 1)

Beispiel 4

5-Hydroxy-N,N-diethyl-1-sulfonsäureamid

24,7 g (62,1 mmol) Bis-(N,N-Diethyl)naphthalin-1,5-disulfonsäureamid werden mit 250 ml 8 %iger Natronlauge verrührt. Die Suspension wird bei 230° C (24 bar) 5 Stunden gerührt. Nach dem Abkühlen ist das Produkt teilweise ausgefallen, wird abgesaugt und getrocknet. Da das Rohprodukt noch ca. 60 % Ausgangsprodukt enthält, wird es in 500 ml 8 %iger NaOH suspendiert, von Ungelöstem abgesaugt und das Filtrat mit konzentrierter Salzsäure auf pH 5 gestellt, dabei fällt das Produkt aus. Die Kristallisation wird durch Abkühlen vervollständigt, das Produkt abgesaugt und getrocknet; 4,44 g (25,6 % der Theorie; 65,3 % bezogen auf umgesetztes Ausgangsprodukt). Durch Verlängerung der Reaktionszeit auf 20 Stunden wird vollständige Umsetzung erreicht.

Beispiel 5

5-Hydroxy-1-naphthalin-sulfonsäureanilid

14,3 g (32,6 mmol) Naphthalin-1,5-disulfonsäureanilid werden mit 250 ml 20 %iger Natronlauge 15 Stunden lang bei 230° C (25 bar) im Autoklaven gerührt. Nach dem Erkalten wird abgesaugt. Der kristalline Rückstand besteht aus 3,67 g Ausgangsprodukt. Dann wird mit konzentrierter Salzsäure auf pH 5 gestellt, worauf das Produkt ausfällt, man rührt noch einige Zeit um die Kristallisation zu vervollständigen, saugt ab und trocknet; 5,19 g (71,5 % der Theorie bezogen auf umgesetztes Ausgangsprodukt).
NMR (siehe Tabelle 1)

Beispiel 6

5-Hydroxy-N,N-dimethyl-1-naphthalinsulfonsäureamid

17,1 g (50,0 mml) Bis-(N,N-Dimethyl)-naphthalin-1,5-disulfonsäureamid werden mit 250 ml 20 %iger Natronlauge suspendiert und 10 Stunden lang im Autoklaven bei 230° C gerührt. Nach dem Abkühlen wird die Reaktionsmischung durch Filtration von Verunreinigungen geklärt und mit 400 ml Wasser verdünnt.
Dann wird mit 25 %iger wäßriger Schwefelsäure auf pH 4 gestellt. Der kristalline Niederschlag wird abgesaugt, mit wenig Wasser gewaschen und getrocknet, 6,40 g Rohprodukt, aus der Mutterlauge werden weitere 1,58 g erhalten, zusammen 7,98 g (63,6 % der Theorie).
NMR (siehe Tabelle 1) (vgl. DE-PS 463 800).

Tabelle 1

$^1$H-NMR-Daten von 5-Hydroxy-1-naphthalin-sulfonamiden (in $[D]_6$DMSO, Tetramethylsilan als interner Standard)

| Bsp. | $R_1$ | $R_2$ | 2-H | 3-H | 4-H | 6-H | 7-H | 8-H | Sonstige |
|---|---|---|---|---|---|---|---|---|---|
| 1 | H | H | 6,99(d) | 7,44(t) | 8,01(d) | 8,36(d) | 7,49(t) | 8,07(d) | 7,5 ($NH_2$)<br>10,4 (OH) |
| 3 | H | Me | 7,00(d) | 7,44(t) | 7,94(d) | 8,46(d) | 7,50(t) | 8,03(d) | 2,40 ($NHCH_3$) |
| 6 | Me | Me | 6,99(d) | 7,45(t) | 8,04(d) | 8,46(d) | 7,55(t) | 8,04(d) | 2,74<br>($-N(CH_3)_2$) |
| 4 | Et | Et | 6,99(d) | 7,45(t) | 7,90(d) | 8,43(d) | 7,51(t) | 8,02(d) | 0,99<br>(t, $2CH_3$)<br>3,27<br>(q, $2CH_2N$) |
| 5 | H | Ph | 6,90(m) | 7,05(t) | 8,23(d) | 8,47(d) | 7,45(t) | 8,28(d) | 7,1<br>(m. arom. H). |

## Patentansprüche

**1.** Verfahren zur Herstellung von Hydroxynaphthalinsulfonsäureamiden der Formel

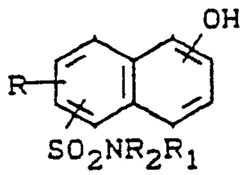

worin

R, $R_1$, $R_2$ = H oder Substituent

dadurch gekennzeichnet, daß man Disulfonamide der Formel

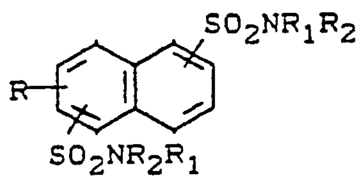

worin

R, $R_1$, $R_2$ = H, Substituent

bei etwa 180 bis 250° C mit Alkalien behandelt.

2. Verfahren gemäß Anspruch 1, dadurch gekennzeichnet, daß die Umsetzung bei etwa 180 bis 250° C mit Natrium- oder Kaliumhydroxid in wäßrigem Medium erfolgt.

3. Verfahren nach Ansprüchen 1 und 2, dadurch gekennzeichnet, daß etwa 1 bis 10 Mol Alkali verwendet werden.

## Claims

1. Process for the preparation of hydroxynaphthalene-sulphonamides of the formula

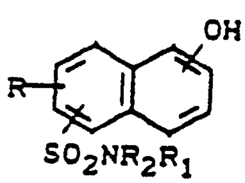

in which
R, $R_1$ and $R_2$ = H or a substituent,
characterised in that disulphonamides of the formula

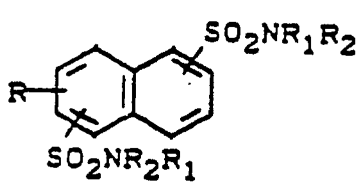

in which
R, $R_1$ and $R_2$ = H or a substituent,
are treated with alkalis at about 180 to 250° C.

2. Process according to Claim 1, characterised in that the reaction is carried out in aqueous medium at about 180 to 250° C using sodium hydroxide or potassium hydroxide.

3. Process according to Claims 1 and 2, characterised in that about 1 to 10 mol of alkali are used.

## Revendications

1. Procédé pour la préparation d'amides d'acides hydroxynaphtalènesulfoniques de formule

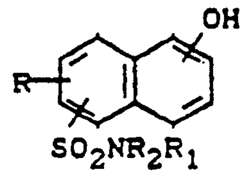

dans laquelle
R, $R_1$, $R_2$ = H ou un substituant,
caractérisé en ce que l'on traite des disulfonamides de formule

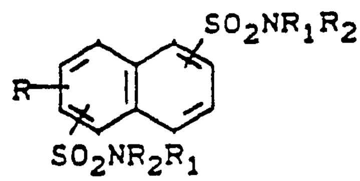

dans laquelle
R, $R_1$, $R_2$ = H ou un substituant
par des alcalis à environ 180-250° C.

2. Procédé selon la revendication 1, caractérisé en ce que la réaction est effectuée à environ 180 à 250° C avec l'hydroxyde de sodium ou de potassium en milieu aqueux.

3. Procédé selon les revendications 1 et 2, caractérisé en ce que l'on utilise environ 1 à 10 mol d'alcali.